# EUROPÄISCHE PATENTANMELDUNG

(11) **EP 3 599 464 A2**
(43) Veröffentlichungstag der Anmeldung: **29.01.2020**
(21) Anmeldenummer: 19184543.7
(22) Anmeldetag: 04.07.2019
(51) Int. Cl.: G01N 33/18

(54) **EINRICHTUNG ZUR BESTIMMUNG EINER EIGENSCHAFT VON WASSER**

(30) Priorität: 25.07.2018 DE 102018005846
(71) Anmelder: Diehl Metering GmbH, 91522 Ansbach (DE)
(72) Erfinder: Aicher, Konrad, 90475 Nürnberg (DE)
(74) Vertreter: Diehl Patentabteilung

(57) **Zusammenfassung**

Einrichtung zur Bestimmung wenigstens einer chemischen oder biologischen oder physikalischen Eigenschaft eines durch eine Kaffeemaschine (1) gelaufenen Wassers (23), umfassend ein zum Einsetzen in die Kaffeemaschine (1) geeignetes Behältnis (3) beinhaltend ein Mittel (4), das mit dem die Kaffeemaschine (1) durchlaufenden Wasser in Kontakt kommt und in Abhängigkeit der Eigenschaft mit dem Wasser reagiert, wobei ein Bestimmungsmittel (5, 17) zur Bestimmung der Eigenschaft anhand einer durch die Reaktionsprodukte veränderbaren Trübung und/oder Färbung des durchgelaufenen Wassers (23) vorgesehen ist.

## Beschreibung

Die Erfindung betrifft eine Einrichtung zur Bestimmung wenigstens einer chemischen oder biologischen oder physikalischen Eigenschaft eines durch eine Kaffeemaschine gelaufenen Wassers.

Bei Lebensmitteln wie Trinkwasser sollte stets ein möglichst hoher Qualitätsstandard sichergestellt sein, um insbesondere die Gesundheit der Verbraucher zu schützen. Besonders relevant hierbei sind mögliche Keimbelastungen und/oder die Konzentration gesundheitsgefährdender Stoffe im Trinkwasser. Aus diesem Grund werden regelmäßig Kontrollen seitens zentraler Verteilungsstellen des Trinkwassernetzes durchgeführt. Besonderes Augenmerk wird dabei auf die Einhaltung entsprechender gesetzlich vorgegebener Grenzwerte gelegt. Durch die regelmäßigen Kontrollen kann zwar sichergestellt werden, dass das bereitgestellte Trinkwasser die gesetzlichen Grenzwerte einhält, allerdings erfolgen Beeinträchtigungen der Trinkwasserqualität oft erst im Bereich des Endverbrauchers. Derartige Beeinträchtigungen der Trinkwasserqualität können aber letztendlich erst bei der endverbraucherseitigen Entnahmestelle festgestellt werden.

Dies zusammen mit einem immer stärker werdenden Gesundheitsbewusstsein in der Bevölkerung führt zu dem Bedürfnis, Trinkwasser auch häuslich hinsichtlich der Trinkwasserqualität überprüfen zu können. Zudem ist es vor allem auch in gastronomischen Betrieben besonders vorteilhaft, wenn eine ausreichend hohe Wasserqualität durch laufende Kontrollen vor Ort gewährleistet ist. Übliche Messverfahren zur Bestimmung der insbesondere verbraucherseitigen Trinkwasserqualität sind allerdings meist sehr aufwendig. Oft müssen zu diesem Zweck Wasserproben an den Zapfstellen entnommen werden, die anschließend an entsprechend eingerichtete Labors geschickt werden, was zu einem nicht unerheblichen Aufwand an Zeit und Kosten führt.

Vor diesem Hintergrund stellt sich die Aufgabe, eine Möglichkeit für eine einfache bzw. mit möglichst wenig Aufwand verbundene, aber dennoch zuverlässige Analyse der Wasserqualität anzugeben.

Zur Lösung dieses Problems ist erfindungsgemäß vorgesehen, dass eine Einrichtung der eingangs genannten Art ein zum Einsetzen in die Kaffeemaschine geeignetes Behältnis beinhaltend ein Mittel, das mit dem die Kaffeemaschine durchlaufenden Wasser in Kontakt kommt und in Abhängigkeit der Eigenschaft mit dem Wasser reagiert, umfasst, wobei ein Bestimmungsmittel zur Bestimmung der Eigenschaft anhand einer durch die Reaktionsprodukte veränderbaren Trübung und/oder Färbung des durchlaufenden Wassers vorgesehen ist.

Dadurch, dass ein Nutzer zur Bestimmung der Wasserqualität bzw. der Eigenschaft zunächst lediglich das Behältnis der erfindungsgemäßen Einrichtung in eine in vielen Haushalten ohnehin bereits vorhandene Kaffeemaschine einsetzen muss, ist der vorbereitende Aufwand zur Bestimmung der Wasserqualität gering. Insbesondere entfällt bei der vorliegenden Erfindung die Notwendigkeit, Wasserproben manuell zu entnehmen, da Kaffeemaschinen üblicherweise direkt an der Wasserleitung angeschlossen sind oder einen mit Leitungswasser gefüllten Wassertank aufweisen.

Die Handhabung der erfindungsgemäßen Einrichtung zusammen mit der Kaffeemaschine ist aus Sicht des Nutzers ähnlich wie die Zubereitung eines Kaffees und mithin für den Nutzer denkbar einfach. Erfindungsgemäß wird ein Mittel verwendet, das gezielt eine definierte, insbesondere chemische, und eigenschaftsbezogene Reaktion mit dem Wasser, das die Kaffeemaschine durchläuft, hervorruft, wobei die Reaktionsprodukte die Änderung der Trübung und/oder Färbung des Wassers verursachen. Definiert bedeutet hier, dass der Zusammenhang zwischen der zu bestimmenden Eigenschaft und der Stärke der Reaktion, also folglich auch zwischen der zu bestimmenden Eigenschaft und der Stärke der Trübung und/oder Färbung, hinlänglich bekannt ist. Dadurch wird anhand der Änderung die zu bestimmende Eigenschaft des Wassers durch das Messmittel bestimmbar. Als Beispiel hierfür sei das Mittel Phenolphthalein zur Bestimmung des pH-Wertes des Wassers genannt. Beim Durchlaufen des Wassers durch die Maschine kommt das Wasser mit dem Phenolphthalein in Kontakt. Eine daraufhin stattfindende Reaktion zwischen dem Phenolphthalein und dem Wasser führt zu einem Farbumschlag des Wassers. Da hinlänglich bekannt ist, welche Farbe sich in Abhängigkeit des pH-Wertes des Wassers einstellt, kann dies genutzt werden, den pH-Wert des Wassers zu bestimmen. Dazu wird erfindungsgemäß das Messmittel genutzt, auf das weiter unten noch detailliert eingegangen wird. Das Mittel ist folglich spezifisch im Hinblick auf die zu ermittelnde Eigenschaft bezüglich des Reaktionsverhaltens gewählt.

Ein Vorteil hierbei ist, dass die Erzeugung des getrübten bzw. verfärbten Wassers unter stets gleich bleibenden und bekannten Bedingungen erfolgt. Vor allem ist sichergestellt, dass das Volumen und die Temperatur des Wassers, das die Kaffeemaschine durchläuft, stets bekannt und definiert sind. Dadurch können Messfehler, die aufgrund unterschiedlicher Messbedingungen auftreten könnten, minimiert werden.

Schließlich entfällt bei der erfindungsgemäßen Einrichtung die Notwendigkeit, entsprechend eingerichtete Labors in die Bestimmung der Qualität des Wassers miteinbeziehen zu müssen. Die Bestimmung der Wasserqualität kann stattdessen im häuslichen Bereich des Nutzers durch eine Analyse des getrübten und/oder verfärbten Wassers durch das Bestimmungsmittel erfolgen. Mithin werden Laufzeiten vermieden, die sich ansonsten durch die postalische Versendung von Wasserproben an Labors und die zugehörige Zurücksendung der Testergebnisse ergeben. Ein unverzügliches Handeln im Fall problematischer Messergebnisse ist hierbei möglich.

Das Behältnis der erfindungsgemäßen Einrichtung kann ein Pad oder eine Kapsel sein. Da bei den meisten handelsüblichen Kaffeemaschinen ohnehin eine Nutzung von mit Kaffeepulver oder dergleichen gefüllten Pads oder Kapseln vorgesehen ist, ist es zweckmäßig, ein dementsprechendes Behältnis beinhaltend das Mittel zu nutzen. Die Hülle des Pads kann, ähnlich wie bei einem Teebeutel, aus einem wasserdurchlässigen Zellstoff oder dergleichen bestehen. Hingegen bestehen Kaffeekapseln üblicherweise aus flüssigkeitsundurchlässigen Materialien wie Kunststoffe und/oder Aluminium, wobei die Kapsel durchstochen werden muss, so dass das durch die Kaffeemaschine laufende Wasser mit dem Inhalt der Kapsel in Kontakt kommen kann. Während bei einem flüssigen Mittel die Verwendung einer Kapsel vorteilhaft ist, ist ansonsten die Ausführungsform, bei der das Behältnis ein Pad ist, aus ökologischer Sicht vorteilhaft.

Bei der erfindungsgemäßen Einrichtung kann vorgesehen sein, dass die Eigenschaft eine Wasserhärte und/oder eine Keimbelastung und/oder ein Salzgehalt und/oder ein pH-Wert und/oder ein Nitratgehalt und/oder ein Chlorgehalt des Wassers ist. Insbesondere die Bestimmung von Eigenschaften, die eine Gefährdung der Gesundheit bei einer Überschreitung des jeweiligen Grenzwertes mit sich bringen würde, ist zweckmäßig. In diesem Zusammenhang sei insbesondere die Belastung des Trinkwassers durch Keime oder Nitrat genannt. Bezüglich der Keimbelastung kann beispielsweise die Belastung durch Legionellen ermittelt werden, wobei diese insbesondere bei Warmwasser auftretenden Erreger die nicht ungefährliche Legionärskrankheit hervorrufen können. Auch die Bestimmung einer eventuellen Schmermetallbelastung des Wassers, die insbesondere bei älteren Wasserleitungen aus Blei auftreten kann, durch die erfindungsgemäße Einrichtung ist denkbar.

Weiterhin kann vorgesehen sein, dass das Mittel Quecksilber(II)-thiocyanat zur Bestimmung des Chloridgehalts oder Kaliumiodat zur Bestimmung des Sulfidgehalts oder Ammoniumvanadat bzw. Ammoniumheptamolybdat zur Bestimmung des Phosphatgehalts oder Methylorange bzw. Phenolphthalein zur Bestimmung des pH-Werts verwendet wird. In diesem Zusammenhang ist auch denkbar, dass das Behältnis eine Mischung aus zwei oder mehreren dieser oder weiterer Stoffe beinhaltet. Daraus ergibt sich der Vorteil, dass im Rahmen eines einzigen Durchlaufens von Wasser durch die Kaffeemaschine mehr als nur eine Eigenschaft bestimmt werden kann.

Erfindungsgemäß kann eine Aufnahmevorrichtung zur Aufnahme des durch die Kaffeemaschine gelaufenen Wassers vorgesehen sein, wobei das Bestimmungsmittel an oder in der Aufnahmevorrichtung angeordnet ist. Die Aufnahmevorrichtung kann insbesondere ein Gefäß sein, das, wie eine Kaffeetasse, an den Auslassstutzen der Kaffeemaschine zur Aufnahme des durch die Kaffeemaschine gelaufenen Wasser gestellt wird. In einer vorteilhaften Ausführung der Erfindung ist das Messmittel derart an oder in der Aufnahmevorrichtung angeordnet, dass das durch die Kaffeemaschine gelaufene Wasser in Kontakt mit dem Bestimmungsmittel kommt, sobald es in der Aufnahmevorrichtung aufgenommen wird. Daraus ergibt sich der Vorteil, dass der Nutzer weder eine separate Tasse oder dergleichen zur Aufnahme des durch die Kaffeemaschine gelaufenen Wassers bereitstellen muss, noch anschließend selbst mit dem Messmittel hantieren muss. Dies stellt eine weitere Vereinfachung für den Nutzer dar.

Zudem ist denkbar, dass das Bestimmungsmittel eine Farbskala ist oder aufweist, wobei die Eigenschaft anhand eines visuellen Vergleichs des durch die Kaffeemaschine gelaufenen Wassers mit der Farbskala durch einen Nutzer bestimmbar ist. Die Verwendung einer Farbskala ist insbesondere dann sinnvoll, wenn das Mittel eine Verfärbung des durch die Kaffeemaschine gelaufenen Wassers bewirkt. Der Begriff Farbskala umfasst im Zusammenhang mit der vorliegenden Erfindung auch eine Skala, die verschiedene Graustufen, die mit dem Wasser verglichen werden können, aufweist. Die Farbskala kann dabei auf einem Papier oder Kunststoffstreifen aufgedruckt sein, der mit der Farbe des durch die Kaffeemaschine gelaufenen Wassers verglichen wird.

Um die Bestimmung der Eigenschaft für den Nutzer zu vereinfachen, können entlang der Farbskala zu den jeweiligen Farben korrespondierende Werte bezüglich der durch die Farbskala wiedergegebenen Eigenschaft bzw. des Qualitätsparameters des Wassers angegeben bzw. aufgedruckt sein. Als Beispiel sei hierzu das Mittel Methylorange zur Bestimmung des pH-Werts genannt. Es ist denkbar, dass die Farbskala die für Methylorange typischen Farbwerte von rot bis gelb umfasst, wobei benachbart zu bzw. entlang der Skala die zu den jeweiligen Farbwerten korrespondierende pH-Werte dargestellt sind. Für eine Skala zur Bestimmung der Härte des Wassers ist denkbar, dass entlang dieser Skala die mit den jeweiligen Farbwerten korrespondierenden Härtegrade dargestellt sind. Dies stellt für den Nutzer eine denkbar einfache Möglichkeit dar, den Qualitätsparameter des Wassers mit geringem Aufwand zu bestimmen.

In einer bevorzugten Ausführungsform kann die Farbskala zusammen mit den korrespondierenden Werten auf der Aufnahmevorrichtung aufgedruckt sein. Die Farbskala mit den Werten kann in dem Bereich der tassenförmigen Aufnahmevorrichtung vorgesehen sein, in dem auch das durch die Kaffeemaschine durchgelaufene Wasser aufgenommen ist. Dies ist vor allem dann sinnvoll, wenn die Aufnahmevorrichtung aus einem undurchsichtigen Kunststoff oder Porzellan oder dergleichen besteht. Die Farbskala kann dann zweckmäßigerweise im Bereich des Wasserspiegels in der Aufnahmevorrichtung vorgesehen sein. Für den Fall, dass die Aufnahmevorrichtung aus einem transparenten Kunststoff oder Glas oder dergleichen besteht, ist denkbar, dass die Farbskala mit den Werten außen auf der Vorrichtung, insbesondere unterhalb des Wasserspiegels, aufgedruckt ist. die Ein separater Vergleichsstreifen oder dergleichen ist bei diesen Ausführungsformen nicht nötig.

In einer weiteren bevorzugten Ausführungsform der Erfindung kann vorgesehen sein, dass ein ein Maß für die Eigenschaft darstellender Messwert betreffend das durch die Kaffeemaschine gelaufene Wasser durch eine Sensoreinrichtung des Bestimmungsmittels erfassbar und an eine Steuerungseinrichtung des Bestimmungsmittels übertragbar ist, wobei die Steuerungseinrichtung dazu eingerichtet ist, die Eigenschaft anhand des Messwertes zu bestimmen. In diesem Fall erfasst die Sensoreinrichtung einen die Trübung und/oder die Verfärbung des Wassers betreffenden Messwert, anhand dessen die Eigenschaft des Wassers bestimmbar ist. Besonders vorteilhaft kann die Bestimmung des Messwertes bzw. der Eigenschaft des Wassers ohne weiteres Zutun des Nutzers, mithin automatisch, gestartet werden. Dadurch können von dem Nutzer verursachte Messunsicherheiten ausgeschlossen werden.

Die Sensoreinrichtung kann ein optischer Sensor wie beispielsweise eine Kamera, ein Photodetektor, ein Spektrometer oder dergleichen sein. Um definierte Messbedingungen zu gewährleisten, kann das Bestimmungsmittel zudem eine Lichtquelle, insbesondere eine Weißlichtlampe oder einen Laser oder dergleichen, umfassen. Bei der Erfassung des Messwerts durch die Sensoreinrichtung kann die Lichtquelle zweckmäßig eingeschaltet sein, wodurch definierte Beleuchtungsverhältnisse bei dem verfärbten und/oder getrübten Wasser sichergestellt werden. Zudem können an der Lichtquelle und/oder der Sensoreinrichtung optische Filter zur Generierung und/oder Erfassung von Licht in definierten Wellenlängenbereichen vorgesehen sein.

Die Sensoreinrichtung und gegebenenfalls die Lichtquelle können in oder an einem Ring aus insbesondere Kunststoff angeordnet sein, der, sofern die Aufnahmevorrichtung aus einem transparenten Material besteht, außen an der Aufnahmevorrichtung befestigt ist. Alternativ kann die Sensoreinrichtung auch direkt an der Aufnahmevorrichtung angeordnet sein.

Trübung kann im Rahmen der vorliegenden Erfindung bedeuten, dass bei einem das Wasser durchlaufenden Lichtstrahl aufgrund mikroskopischer, die Trübung verursachender, Partikel eine diffuse Seitwärtsstreuung auftritt. Alternativ oder zusätzlich kann die Trübung eine Veränderung der Absorptionseigenschaften des Wassers bezüglich des das Wasser durchlaufenden Lichtstrahls bewirken.

Sofern die Sensoreinrichtung ein Photodetektor ist, kann der Messwert ein in Abhängigkeit der in den Photodetektor einfallenden Lichtintensität elektrisches Signal und/oder einen veränderlichen elektrischen Widerstand des Photodetektors betreffen. Zu diesem Zweck ist denkbar, dass ein von der Lichtquelle emittierter Lichtstrahl eine, insbesondere durch die geometrische Ausgestaltung der Aufnahmevorrichtung definierte Wegstrecke durch das getrübte und/oder verfärbte Wasser durchläuft. Es ist denkbar, dass der das Wasser durchlaufende Lichtstrahl direkt auf den Photodetektor trifft. Zusätzlich oder alternativ kann der oder ein weiterer Photodetektor den Messwert anhand des seitwärts gestreuten Anteil des Lichtstrahls ermitteln.

Zudem ist denkbar, dass die Sensoreinrichtung eine ein Bild des beleuchteten Wassers aufnehmende Kamera ist oder umfasst, wobei der Messwert beispielsweise die durchschnittlichen Farbkoordinaten der Bildpixel des aufgenommenen Bildes betrifft, anhand dessen die Eigenschaft des Wassers bestimmbar ist.

Im Zusammenhang mit der Erfassung des Messwerts kann vorgesehen sein, dass vor und/oder nach der Messung eine Kalibrations- bzw. Referenzmessung bei der leeren Aufnahmevorrichtung durchgeführt wird. Eventuelle Schwankungen der Lichtleistung der Lichtquelle oder dergleichen können in diesem Fall bei der Bestimmung der Eigenschaft anhand des Messwerts berücksichtigt werden.

Es kann zudem vorgesehen sein, dass während eines Messvorgangs gleichzeitig mehrere Messwerte erfasst werden, die beispielsweise sowohl die Farbe als auch die Trübung des Wassers betreffen. Dabei ist insbesondere denkbar, dass das Behältnis eine Mischung aus mehreren Mitteln oder gegebenenfalls mehrere Kompartimente mit jeweils verschiedenen Mitteln aufweist, wobei das damit in Kontakt kommende Wasser sowohl getrübt als auch verfärbt wird. Zweckmäßigerweise kann die Sensoreinrichtung mehrere Sensoren zur insbesondere gleichzeitigen Erfassung mehrerer Messwerte umfassen.

Zur Bestimmung der Eigenschaft anhand des Messwerts kann vorgesehen sein, dass beispielsweise auf eine geeignete Look-up-Tabelle zurückgegriffen wird. Auch die Verwendung empirischer Modelle ist für diesen Zweck denkbar.

Außerdem kann eine Bedienvorrichtung vorgesehen sein, durch die der Nutzer die Erfassung des Messwertes und mithin die Bestimmung der Eigenschaft starten kann. Insbesondere ist denkbar, dass die Bedienvorrichtung einen Startknopf zum Starten der Messung aufweist. Die Bedienvorrichtung kann bevorzugt an der Aufnahmevorrichtung vorgesehen sein. Zudem ist allerdings auch denkbar, dass die Erfassung des Messwertes automatisch gestartet wird. Hierbei kann ein Feuchtigkeitssensor vorgesehen sein, wobei bei der Aufnahme von Wasser in der Aufnahmevorrichtung ein entsprechendes Signal des Feuchtigkeitssensors von der Steuerungseinrichtung detektiert werden kann, woraufhin die Erfassung des Messwertes automatisch gestartet wird.

Weiterhin ist denkbar, dass eine Anzeigeeinrichtung zur Anzeige der Eigenschaft anhand eines von der Steuerungseinrichtung erzeugten Signals vorgesehen ist. Mit anderen Worten wird dem Nutzer, sobald die Erfassung des Messwertes und die entsprechende Bestimmung der Eigenschaft erfolgt ist, die Eigenschaft an der Anzeigeeinrichtung angezeigt. Daraus ergibt sich der Vorteil, dass der Nutzer die Eigenschaft bequem von der Anzeigeeinrichtung ablesen kann.

Die Bedienvorrichtung und/oder die Anzeigeeinrichtung können direkt an der Aufnahmevorrichtung oder dem mit der Aufnahmeeinrichtung verbundenen Ring aus insbesondere Kunststoff angeordnet sein.

Als eine Weiterbildung hiervon ist denkbar, dass durch die Anzeigeeinrichtung zusätzlich eine Information ausgegeben wird, die ein mögliches Überschreiten eines insbesondere vorab gespeicherten Grenzwertes betrifft. So kann in dem Fall, dass eine Überschreitung eines gesetzlichen Grenzwertes vorliegt, die Eigenschaft über die Anzeigeeinrichtung zusammen mit einem entsprechenden Warnhinweis ausgegeben werden. Der Warnhinweis kann beispielsweise darin bestehen, dass die Eigenschaft in roter Farbe und gegebenenfalls blinkend angezeigt wird. Ein manueller Vergleich der angezeigten Eigenschaft mit festen Grenzwerten durch den Nutzer entfällt hierbei, wodurch der Nutzer mithin keinerlei weitere Kenntnisse benötigt, um sich einer möglichen Überschreitung eines Grenzwertes bewusst zu werden.

Erfindungsgemäß kann vorgesehen sein, dass das Behältnis eine von einem Sensor der Kaffeemaschine erfassbare Information, insbesondere einen Barcode, aufweist, wobei das Volumen und/oder die Temperatur des die Kaffeemaschine durchlaufenden Wassers in Abhängigkeit der Information einstellbar ist. Hierbei kann vorgesehen sein, dass nach Einsetzen des Behältnisses in die Kaffeemaschine z.B. der Barcode automatisch erfasst und ausgewertet wird. Dabei ist denkbar, dass bei der Verwendung unterschiedlicher Messmittel die für das jeweils momentan verwendete Messmittel optimalen Wassertemperatur und/oder das optimale Wasservolumen verwendet werden. Der bei dieser Ausführungsform z.B. verwendete Barcode kann beispielsweise ein EAN-Code sein.

Bezüglich des Volumens des die Kaffeemaschine durchlaufenden Wassers ist denkbar, dass dieses derart eingestellt wird, dass nach Durchlaufen des kompletten Volumens kein Rückstand des Mittels mehr in der Kaffeemaschine verbleibt bzw. automatisch eine Spülung durchgeführt wird. Dadurch kann sichergestellt werden, dass ein möglicherweise nach Erfassung eines Qualitätsparameters zubereiteter Kaffee keine Rückstände des Mittels mehr aufweist. Andernfalls könnte dies z.B. den Geschmack des Kaffees negativ beeinflussen.

Die Erfindung betrifft zudem ein Verfahren zur Bestimmung wenigstens einer chemischen oder biologischen oder physikalischen Eigenschaft eines Wassers, wozu eine vorstehend beschriebene Einrichtung verwendet wird, umfassend die folgenden Schritte:
- Einsetzen eines Behältnisses umfassend ein Mittel in eine Kaffeemaschine,
- Durchlaufen des Wassers durch die Kaffeemaschine, wobei das Mittel mit dem Wasser in Kontakt kommt und in Abhängigkeit der Eigenschaft mit dem Wasser reagiert, und
- Bestimmung der Eigenschaft anhand einer durch die Reaktionsprodukte veränderbaren Trübung und/oder Färbung des durchgelaufenen Wassers durch ein Bestimmungsmittel.

Für das erfindungsgemäße Verfahren kann vorgesehen sein, dass die Eigenschaft eine Wasserhärte und/oder eine Keimbelastung und/oder ein Salzgehalt und/oder ein pH-Wert und/oder ein Nitratgehalt und/oder ein Chlorgehalt des Wassers ist.

Ferner ist für das erfindungsgemäße Verfahren denkbar, dass als Mittel Quecksilber(II)-thiocyanat oder Kaliumiodat oder Ammoniumvanadat oder Ammoniumheptamolybdat oder Methylorange oder Phenolphthalein verwendet wird.

Beim erfindungsgemäßen Verfahren kann vorgesehen sein, dass das durch die Kaffeemaschine durchgelaufene Wasser in einer Aufnahmevorrichtung aufgenommen wird, wobei das Bestimmungsmittel an oder in der Aufnahmevorrichtung angeordnet ist.

Zudem kann für das erfindungsgemäße Verfahren vorgesehen sein, dass eine Sensoreinrichtung des Bestimmungsmittels einen ein Maß für die Eigenschaft darstellenden Messwert betreffend das durchgelaufene Wasser erfasst und an eine Steuerungseinrichtung des Bestimmungsmittels überträgt, wobei die Steuerungseinrichtung die Eigenschaft anhand des Messwertes bestimmt.

Bei dem erfindungsgemäßen Verfahren kann ein Sensor der Kaffeemaschine eine Information, insbesondere einen Barcode, des Behältnisses erfassen und in Abhängigkeit der Information das Volumen und/oder die Temperatur des die Kaffeemaschine durchlaufenden Wassers eingestellt werden.

Sämtliche für die erfindungsgemäße Einrichtung beschriebenen Merkmale sind entsprechend mit den genannten Vorteilen auch auf das erfindungsgemäße Verfahren übertragbar.

Weitere Vorteile und Einzelheiten der Erfindung ergeben sich aus dem im Folgenden beschriebenen Ausführungsbeispiel sowie anhand der Zeichnungen. Dabei zeigen schematisch:
- Fig. 1: eine Ansicht auf eine Kaffeemaschine mit einem Ausführungsbeispiel einer erfindungsgemäßen Einrichtung,
- Fig. 2: eine geschnittene Ansicht eines Behältnisses der erfindungsgemäßen Einrichtung aus Fig. 1, und
- Fig. 3: eine geschnittene Ansicht auf eine Aufnahmevorrichtung der erfindungsgemäßen Einrichtung aus Fig. 1.

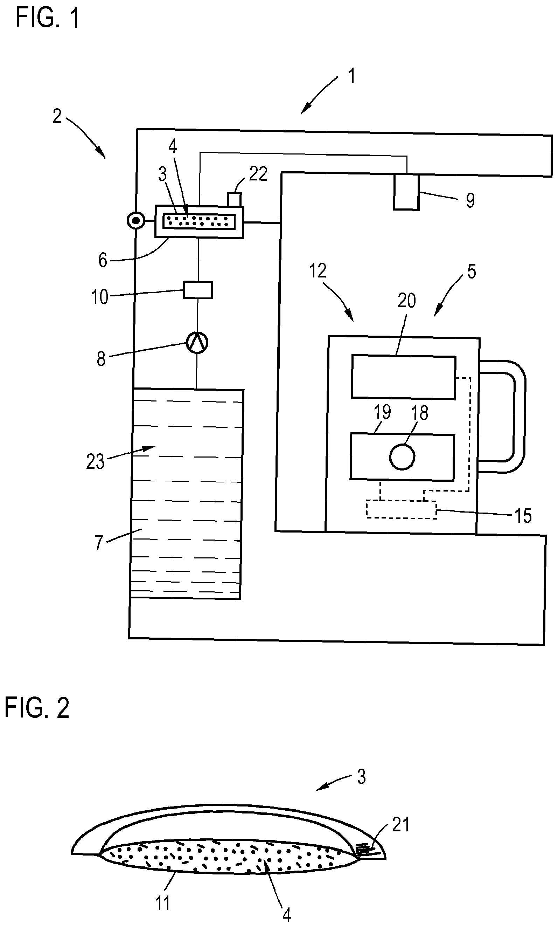

Fig. 1 zeigt schematisch eine Ansicht auf eine Kaffeemaschine 1 zusammen mit einer Einrichtung 2, durch die eine Eigenschaft betreffend die Qualität eines durch die Kaffeemaschine 1 gelaufenen Wassers 23 bestimmt werden kann. Die Einrichtung 2 umfasst ein beispielsweise als Pad ausgebildetes Behältnis 3 (vgl. Fig. 2), das ein Mittel 4 beinhaltet, das in Abhängigkeit einer Eigenschaft betreffend ein Qualitätsmerkmal des Wassers 23 mit dem Wasser 23 reagiert, sobald es damit in Kontakt kommt. Die Einrichtung 2 umfasst ferner ein Bestimmungsmittel 5 zur Bestimmung der Eigenschaft des Wassers anhand einer durch die Reaktionsprodukte veränderbaren Trübung und/oder Färbung des Wassers 23.

Aus Sicht eines Nutzers erfolgt die Bestimmung der Eigenschaft dadurch, dass zunächst das Behältnis 3 in ein Fach 6 der Kaffeemaschine 1 eingesetzt wird. Exemplarisch wird zu diesem Zweck die Kaffeemaschine 1 aufgeklappt, wobei in das Fach 6 üblicherweise Pads zur Herstellung von Kaffee oder anderer Heißgetränke eingesetzt werden. Anschließend wird Wasser 23 aus einem Wassertank 7 oder einer Wasserleitung, an die die Kaffeemaschine 1 angeschlossen ist, über eine Pumpe 8 durch das Behältnis 3 geleitet, wodurch das Wasser 23 mit dem Mittel 4 in Kontakt kommt. Dabei erfolgt eine definierte, insbesondere chemische, Reaktion des Mittels mit dem Wasser 23. Die Reaktionsprodukte führen zu einer Trübung und/oder Färbung des Wassers 23. Dabei ist die Stärke der Reaktion bzw. die Änderung der Trübung und/oder Färbung in Abhängigkeit der zu bestimmenden Eigenschaft bzw. des zu bestimmenden Qualitätsparameters bekannt. Als Beispiel hierfür kann Phenolphthalein genannt werden, wobei hier die zu bestimmende Eigenschaft der pH-Wert des Wassers 23 ist. In Abhängigkeit des pH-Werts stellt sich bei diesem Beispiel ein definierter Farbumschlag des Wassers 23 ein, anhand dessen letztendlich der pH-Wert durch das Bestimmungsmittel 5 bestimmt werden kann. Zu diesem Zweck wird das durch die Kaffeemaschine 1 gelaufene Wasser 23 über einen Auslassstutzen 9 dem Bestimmungsmittel 5 zugeführt, anhand dessen die Eigenschaft durch eine Auswertung der Trübung und/oder Färbung des durch die Kaffeemaschine 1 gelaufenen Wassers 23 bestimmt wird. Eine Heizeinrichtung 10 der Kaffeemaschine 1 kann zudem vorgesehen sein, das kalte Wasser 23 aus dem Wassertank 7 oder der Wasserleitung auf eine gewünschte Temperatur aufzuheizen.

Mithin ist aus Sicht des Nutzers die Handhabung bei der Erzeugung des getrübten und/oder gefärbten Wassers 23 ähnlich zu der Handhabung bei der Zubereitung eines Kaffees. Er muss lediglich anstelle eines Kaffeepads das Behältnis 3 in das Fach einsetzen. Bei der erfindungsgemäßen Einrichtung 2 wird es dem Nutzer mithin ermöglicht, die Wasserqualität direkt zu Hause ohne großen Aufwand und möglichst einfach zu bestimmen. Die umständliche Entnahme von Wasserproben und entsprechende Übersendung dieser Proben an zur Durchführung von Tests zur Bestimmung der Wasserqualität eingerichtete Labors entfällt hierbei. Da es für viele dieser Qualitätsparameter gesetzliche Grenzwerte gibt und derartige Belastungen im Wasserleitungssystem auch häufig erst beim Endverbraucherseitigen erfolgen, ist eine regelmäßige Überprüfung dieser Parameter auch im häuslichen Bereich des Endverbrauchers sowie insbesondere in Gastronomiebetrieben oder bei größeren gastronomischen Veranstaltungen, besonders wünschenswert, was durch die Einrichtung 2 ermöglicht wird.

Eine geschnittene Detailansicht eines exemplarischen Behältnisses 3 ist in Fig. 2 dargestellt. Das Behältnis 3 ist im gezeigten Beispiel ein Kaffeepad, bei dem das pulver- oder granulatförmige Mittel 4 in einer wasserdurchlässigen Zellstoffhülle 11 aufgenommen ist. Ferner ist denkbar, dass das Behältnis 3 eine Kapsel aus Kunststoff oder einem anderen flüssigkeitsundurchlässigen Material ist, wobei die Kapsel vor bzw. bei dem Durchlaufen des Wassers 23 durchstochen wird. Insbesondere für den Fall, dass es sich bei dem Mittel um eine Flüssigkeit handelt, ist die Verwendung einer Kapsel zweckmäßig. Eine Kapsel kann selbstverständlich auch verwendet werden, sofern das Mittel 4 in Form eines Pulvers oder Granulats vorliegt.

Die zu bestimmende Eigenschaft des Wassers 23 kann eine Wasserhärte und/oder eine Keimbelastung und/oder ein Salzgehalt und/oder ein pH-Wert und/oder ein Nitratgehalt und/oder ein Chlorgehalt oder weitere Qualitätsparameter des Wassers 23 wie beispielsweise eine Schwermetallbelastung sein. Das im Behältnis 3 aufgenommene Mittel 4 kann beispielsweise Quecksilber(II)-thiocyanat zur Bestimmung eines Chloridgehaltes, Kaliumiodat zur Bestimmung des Sulfidgehalts, Ammoniumvanadat bzw. Ammoniumheptamolybdat zur Bestimmung eines Phosphatgehalts und/oder Methylorange bzw. Phenolphthalein zur Bestimmung eines pH-Werts des Wassers 23 sein. Es ist zudem denkbar, dass das Behältnis 3 eine Mischung aus mehreren Mitteln beinhaltet, wodurch die gleichzeitige Bestimmung mehrerer Eigenschaften bzw. Qualitätsparameter des Wassers 23 ermöglicht wird.

Die Einrichtung 1 umfasst eine Aufnahmevorrichtung 12, die im gezeigten Ausführungsbeispiel als tassenförmiges Aufnahmegefäß für das die Kaffeemaschine 1 durchlaufene Wasser 23 dient. Hierbei ist das Bestimmungsmittel 5 an bzw. in der Aufnahmevorrichtung 12 angeordnet.

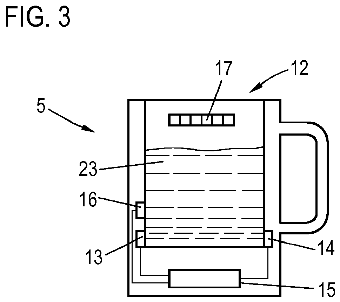

Eine geschnittene Ansicht der exemplarischen Aufnahmevorrichtung 12 ist in Fig. 3 dargestellt. Das Bestimmungsmittel 5 umfasst eine als Lichtsensor bzw. Photosensor ausgebildete Sensoreinrichtung 13 sowie eine optionale Lichtquelle 14. Die Lichtquelle 14 kann ein Laser oder eine Weißlichtquelle sein. Nachdem das durch die Kaffeemaschine gelaufene Wasser 23 in der Aufnahmevorrichtung 12 aufgenommen wurde, erfolgt eine Messung eines Messwerts betreffend das Wasser 23 durch die Sensoreinrichtung 13. Der von der Sensoreinrichtung 13 erfasste Messwert betrifft eine durch das Mittel 4 hervorgerufene Trübung des Wassers 23. Dazu wird eine Lichtquelle 14 eingeschaltet, wobei ein dabei erzeugter Lichtstrahl das in der Aufnahmevorrichtung 12 aufgenommene Wasser 23 entlang einer definierten Wegstrecke durchläuft und auf die als Photodiode ausgebildete Sensoreinrichtung 13 trifft. In Abhängigkeit der Trübung erfolgt eine Abnahme der Intensität des das Wasser 23 durchlaufenden Lichtstrahls, die wiederum indirekt durch einen von der Fotodiode erzeugten elektrischen Impuls erfasst und an eine Steuerungseinrichtung 15 übertragen wird. Anhand dieses Messwertes bestimmt die Steuerungseinrichtung 15 die Eigenschaft des Wassers 23.

Im gezeigten Ausführungsbeispiel ist zusätzlich eine optionale zweite Sensoreinrichtung 16 des Bestimmungsmittels 5 vorgesehen. Die zweite Sensoreinrichtung 16 ist im gezeigten Ausführungsbeispiel eine Kamera zur Erfassung eines Messwerts betreffend die Verfärbung des in der Aufnahmevorrichtung 12 aufgenommenen Wassers 23. Zur Erfassung des Messwertes durch die zweite Sensoreinrichtung 16 wird die Lichtquelle 14 eingeschaltet, wodurch definierte Messverhältnisse erzeugt werden. Der Messwert betrifft nun einen durchschnittlichen Farbwert der Pixel des von der als Kamera ausgebildeten zweiten Sensoreinrichtung 16 erfassten Bildes.

Die Steuerungseinrichtung 15 bestimmt nun anhand der von der Sensoreinrichtung 13 und der zweiten Sensoreinrichtung 16 erfassten Messwerte die Eigenschaft bzw. den Qualitätsparameter des Wassers 23. Zu diesem Zweck sind sowohl die Verwendung numerischer Modelle als auch ein entsprechender Rückgriff auf eine Look-up-Tabelle denkbar. Die Verwendung weiterer Sensoreinrichtungen ist denkbar.

Beim gezeigten Ausführungsbeispiel ist ein optionales weiteres Bestimmungsmittel 17 vorgesehen. Das weitere Bestimmungsmittel 17 ist eine an der Innenseite der Aufnahmevorrichtung 12 vorgesehene Farbskala, wobei unmittelbar neben der Farbskala die zu den jeweiligen Farben korrespondierenden Werte bezüglich der Eigenschaft des Wassers 23 angeordnet sind. Der Nutzer kann dadurch den mit den Farbwerten korrespondierenden Qualitätsparameter des Wassers durch einen visuellen Vergleich des Wassers 23 mit der Farbskala bestimmen. So ist im gezeigten Ausführungsbeispiel eine Bestimmung der mit dem Farbwert des Wassers 23 korrespondierenden Eigenschaft einerseits über die zweite Sensoreinrichtung 16 und andererseits über das weitere Bestimmungsmittel 17 möglich. Der Nutzer hat hierbei die Möglichkeit, den durch die zweite Sensoreinrichtung 16 ermittelten Wert bezüglich des Qualitätsparameters zu verifizieren.

Das Starten der Messung bzw. der Bestimmung der Eigenschaft anhand des gefärbten bzw. getrübten Wassers 23 erfolgt dadurch, indem der Nutzer einen Startknopf 18 einer Bedienvorrichtung 19 drückt. Bei dieser optionalen Bedienvorrichtung 19 kann zudem vorgesehen sein, dass diese weitere Bedienelemente umfasst, wodurch der Nutzer auswählen kann, welche Eigenschaft des Wassers 23 durch das Bestimmungsmittel 5 ermittelt werden soll. Die Bedienvorrichtung 19 ist zu diesem Zweck mit der Steuerungseinrichtung 15 verbunden. Alternativ kann bezüglich des Startens der Messung vorgesehen sein, dass die Aufnahmevorrichtung 12 einen mit der Steuerungseinrichtung 15 verbundenen Feuchtigkeitssensor umfasst, wobei bei Detektion der Aufnahme des Wassers 23 in der Aufnahmevorrichtung 12 der Messvorgang automatisch gestartet wird.

Außerdem umfasst die Einrichtung 1 eine mit der Steuerungseinrichtung 15 verbundene Anzeigeeinrichtung 20, über die dem Nutzer die von der Steuerungseinrichtung 15 ermittelte Eigenschaft angezeigt wird. Im Zusammenhang mit der optionalen Anzeigeeinrichtung 20 ist vorgesehen, dass die Steuerungseinrichtung die ermittelte Eigenschaft des Wassers 23 mit vorgegebenen Grenzwerten vergleicht und im Falle einer Überschreitung des Grenzwertes ein entsprechendes Warnsignal durch die Anzeigeeinrichtung 20 ausgegeben wird. Dieses kann beispielsweise darin bestehen, dass bei Überschreiten des Grenzwertes die betroffene Eigenschaft in roter Farbe und/oder blinkend von der Anzeigeeinrichtung 20 angezeigt wird. Bei Unterschreiten des Grenzwertes kann die Eigenschaft von der Anzeigeeinrichtung in grüner Farbe angezeigt werden.

Zudem ist beim gezeigten Ausführungsbeispiel vorgesehen, dass das Behältnis 3 eine optionale Information, hier einen Barcode 21 aufweist, die bzw. der über einen Sensor 22 der Kaffeemaschine ausgelesen wird, wobei anhand des Barcodes das Volumen des das Behältnis 3 durchlaufenden Wassers 23 sowie die Temperatur eingestellt wird. Auf diese Weise können die Messverhältnisse individuell an ein jeweils verwendetes Mittel 4 angepasst werden. Zudem kann das Volumen des Wassers 23 derart gewählt werden, dass nach dem Durchlaufen des Wasservolumens durch das Behältnis 3 bzw. durch die Kaffeemaschine 1 keine Rückstände des Mittels 4 mehr in der Kaffeemaschine 1 verbleiben, die die Qualität eines danach zubereiteten Kaffees negativ beeinflussen könnten. Zu diesem Zweck ist außerdem beispielsweise vorgesehen, dass nach dem Durchlaufen des Wassers 23 durch die Kaffeemaschine 1 ein automatischer Spülvorgang gestartet wird, bei dem mögliche Reste des Mittels 4 in der Kaffeemaschine 1 ausgespült werden.

### Bezugszeichenliste

- 1: Kaffeemaschine
- 2: Einrichtung
- 3: Behältnis
- 4: Mittel
- 5: Bestimmungsmittel
- 6: Fach
- 7: Wassertank
- 8: Pumpe
- 9: Auslassstutzen
- 10: Heizeinrichtung
- 11: Zellstoffhülle
- 12: Aufnahmevorrichtung
- 13: Sensoreinrichtung
- 14: Lichtquelle
- 15: Steuerungseinrichtung
- 16: Zweite Sensoreinrichtung
- 17: Weiteres Bestimmungsmittel
- 18: Startknopf
- 19: Bedienvorrichtung
- 20: Anzeigeeinrichtung
- 21: Barcode
- 22: Sensor
- 23: Wasser

## Patentansprüche

1. Einrichtung zur Bestimmung wenigstens einer chemischen oder biologischen oder physikalischen Eigenschaft eines durch eine Kaffeemaschine (1) gelaufenen Wassers (23), umfassend ein zum Einsetzen in die Kaffeemaschine (1) geeignetes Behältnis (3) beinhaltend ein Mittel (4), das mit dem die Kaffeemaschine (1) durchlaufenden Wasser in Kontakt kommt und in Abhängigkeit der Eigenschaft mit dem Wasser reagiert, wobei ein Bestimmungsmittel (5, 17) zur Bestimmung der Eigenschaft anhand einer durch die Reaktionsprodukte veränderbaren Trübung und/oder Färbung des durchgelaufenen Wassers (23) vorgesehen ist.

2. Einrichtung nach Anspruch 1, **dadurch gekennzeichnet, dass** das Behältnis (3) ein Pad oder eine Kapsel ist.

3. Einrichtung nach Anspruch 1 oder 2, **dadurch gekennzeichnet, dass** die Eigenschaft eine Wasserhärte und/oder eine Keimbelastung und/oder ein Salzgehalt und/oder ein pH-Wert und/oder ein Nitratgehalt und/oder ein Chlorgehalt des Wassers (23) ist.

4. Einrichtung nach einem der vorangehenden Ansprüche, **dadurch gekennzeichnet, dass** das Mittel (4) Quecksilber(II)-thiocyanat oder Kaliumiodat oder Ammoniumvanadat oder Ammoniumheptamolybdat oder Methylorange oder Phenolphthalein ist.

5. Einrichtung nach einem der vorangehenden Ansprüche, **dadurch gekennzeichnet, dass** eine Aufnahmevorrichtung (12) zur Aufnahme des durch die Kaffeemaschine (1) gelaufenen Wassers (23) vorgesehen ist, wobei das Bestimmungsmittel (5, 17) an oder in der Aufnahmevorrichtung (12) angeordnet ist.

6. Einrichtung nach einem der vorangehenden Ansprüche, **dadurch gekennzeichnet, dass** das Bestimmungsmittel (17) eine Farbskala ist oder aufweist, wobei die Eigenschaft anhand eines visuellen Vergleichs des durch die Kaffeemaschine (1) gelaufenen Wassers (23) mit der Farbskala durch einen Nutzer bestimmbar ist.

7. Einrichtung nach einem der vorangehenden Ansprüche, **dadurch gekennzeichnet, dass** ein ein Maß für die Eigenschaft darstellender Messwert betreffend das durch die Kaffeemaschine (1) gelaufene Wasser (23) durch eine Sensoreinrichtung (13, 16) des Bestimmungsmittels (5) erfassbar und an eine Steuerungseinrichtung (15) des Bestimmungsmittels (5) übertragbar ist, wobei die Steuerungseinrichtung (15) dazu eingerichtet ist, die Eigenschaft anhand des Messwertes zu bestimmen.

8. Einrichtung nach Anspruch 7, **dadurch gekennzeichnet, dass** eine Anzeigeeinrichtung (20) zur Anzeige der Eigenschaft anhand eines von der Steuerungseinrichtung (15) erzeugten Signals vorgesehen ist.

9. Einrichtung nach einem der vorangehenden Ansprüche, **dadurch gekennzeichnet, dass** das Behältnis (3) eine von einem Sensor (22) der Kaffeemaschine erfassbare Information, insbesondere einen Barcode (21), aufweist, wobei in Abhängigkeit der Information das Volumen und/oder die Temperatur des die Kaffeemaschine (1) durchlaufenden Wassers (23) einstellbar ist.

10. Verfahren zur Bestimmung wenigstens einer chemischen oder biologischen oder physikalischen Eigenschaft eines Wassers (23), umfassend die folgenden Schritte:
- Einsetzen eines Behältnisses (3) umfassend ein Mittel (4) in eine Kaffeemaschine (1),
- Durchlaufen des Wassers (23) durch die Kaffeemaschine (1), wobei das Mittel (4) mit dem Wasser (23) in Kontakt kommt und in Abhängigkeit der Eigenschaft mit dem Wasser (23) reagiert, und
- Bestimmung der Eigenschaft anhand einer durch die Reaktionsprodukte veränderbaren Trübung und/oder Färbung des durchgelaufenen Wassers (23) durch ein Bestimmungsmittel (5).

11. Verfahren nach Anspruch 10, **dadurch gekennzeichnet, dass** die Eigenschaft eine Wasserhärte und/oder eine Keimbelastung und/oder ein Salzgehalt und/oder ein pH-Wert und/oder ein Nitratgehalt und/oder ein Chlorgehalt des Wassers (23) ist.

12. Verfahren nach Anspruch 10 oder 11, **dadurch gekennzeichnet, dass** als Mittel (4) Quecksilber(II)-thiocyanat oder Kaliumiodat oder Ammoniumvanadat oder Ammoniumheptamolybdat oder Methylorange oder Phenolphthalein verwendet wird.

13. Verfahren nach einem der Ansprüche 10 bis 12, **dadurch gekennzeichnet, dass** das durch die Kaffeemaschine (1) durchgelaufene Wasser (23) in einer Aufnahmevorrichtung (12) aufgenommen wird, wobei das Bestimmungsmittel (5, 17) an oder in der Aufnahmevorrichtung (12) angeordnet ist.

14. Verfahren nach einem der Ansprüche 10 bis 13, **dadurch gekennzeichnet, dass** eine Sensoreinrichtung (13, 16) des Bestimmungsmittels (5) einen ein Maß für die Eigenschaft darstellenden Messwert betreffend das durchgelaufene Wasser (23) erfasst und an eine Steuerungseinrichtung (15) des Bestimmungsmittels (5) überträgt, wobei die Steuerungseinrichtung (15) die Eigenschaft anhand des Messwertes bestimmt.

15. Verfahren nach einem der Ansprüche 10 bis 14, **dadurch gekennzeichnet, dass** ein Sensor (22) der Kaffeemaschine (1) eine Information, insbesondere einen Barcode (21), des Behältnisses (3) erfasst und in Abhängigkeit der Information das Volumen und/oder die Temperatur des die Kaffeemaschine (1) durchlaufenden Wassers (23) eingestellt wird.
